# DEMANDE DE BREVET EUROPEEN

(11) **EP 2 298 382 A1**
(43) Date de publication de la demande: **23.03.2011**
(21) Numéro de dépôt: 09170730.7
(22) Date de dépôt: 18.09.2009
(51) Int. Cl.: A61M 5/00, A61M 5/14

(54) **Dispositif pour l'injection de produits de contraste**

(71) Demandeur: SMC-Swiss Medical Care S.A., 1004 Lausanne (CH)
(72) Inventeur: Duffour, Hervé, 1004 Lausanne (CH); Neftel, Frédéric, 1005 Lausanne (CH)
(74) Mandataire: Grosfillier, Philippe

(57) **Abrégé**

Dispositif médical pour l'injection de produits de contraste comprenant au moins deux réservoirs, un injecteur et un distributeur disposé de manière à mettre en communication de manière alternée lesdits réservoirs avec ledit injecteur, dispositif médical caractérisé par le fait qu'il comprend des moyens pour assurer ladite communication alternée à une fréquence comprise entre 0.2 et 5 Hz.

## Description

### Domaine de l'invention

Le domaine de l'invention est celui de l'imagerie médicale, obtenue par injection de produits de contrastes et, plus précisément, celui de l'injection de produits de contrastes plus ou moins dilués tels que pour l'imagerie par Rx et scanner CT ou celui de l'imagerie IRM.

### Etat de la technique

On connaît déjà aujourd'hui l'utilisation d'injecteurs pousse-seringues capables d'injecter depuis deux seringues en parallèles un produit de contraste simultanément à une solution saline, ceci afin de diluer le produit de contraste. Les brevets US 7267667, ainsi que US 5911252 décrivent de tels dispositifs.

### Exposé général de l'invention

Dans l'invention, la solution du problème précité consiste à injecter du produit de contraste dans un organe à analyser de telle façon que les concentrations du tel produit de contraste varient au court du temps et/ou de la région de l'organe analysé au moment de l'acquisition des images.

La présente invention présente plusieurs avantages, en particulier de pouvoir réaliser de telles modifications de concentration dans l'organe à un moment précis correspondant à l'acquisition des images, sans avoir recours à deux injecteurs utilisés en parallèles, à un moindre coût et avec un moindre risque (notamment d'erreur et/ou de contamination) pour le patient.

### Exposé détaillé de l'invention

L'invention est décrite plus en détail ci-après au moyen d'exemples illustrés par les figures
Un des objectifs de la présente invention est d'obtenir une dilution du produit de contraste (soit en utilisant deux produits de contrastes différents, soit en utilisant un produit de contraste et une solution saline ou toute autre solution qui n'a pas d'effet sur le contraste au cours de l'examen tel qu'une solution pharmaceutique non iodée) avant l'arrivée dans l'organe cible (par exemple le coeur droit du patient), afin de pouvoir améliorer l'acquisition d'informations relatives à l'anatomie et au fonctionnement de l'organe à analyser (dans le cas du coeur : visualisation du septum, des coronaires, meilleur calcul de la fraction d'éjection du coeur ; sachant que pour se faire le coeur gauche est préférentiellement rempli avec du contraste pendant que le coeur droit est rempli avec une solution de contraste diluée), soit au même moment soit au moment respectif ou les acquisitions des images se font respectivement dans l'organe en question (d'abord le coeur gauche, puis le coeur droit).
Pour ce faire l'invention consiste de préférence à injecter successivement dans la veine du patient des phases de produit de contraste alternées avec des phases d'injection de solution saline ou d'un produit de contraste plus faiblement concentré, ceci afin d'obtenir un mélange entre les différentes phases qui puisse se faire dans le système cardiovasculaire du patient avant l'arrivée dans le coeur ou l'organe destiné à être analysé. L'invention peut être avantageusement utilisée dans l'imagerie par Scanner CT ou dans l'IRM. Pour obtenir une dilution efficace il faut que la fréquence soit suffisamment rapide afin que le mélange puisse se faire dans le système cardiovasculaire du patient avant l'arrivée dans l'organe visé. Pour le coeur, il est souhaitable d'avoir une fréquence assez proche de celle de la fréquence cardiaque, typiquement de l'ordre de 1 Hz, mais cela peut également être efficace entre 5Hz et 0.2 Hz.
Ainsi on peut se passer d'un double injecteur et réaliser la dilution directement dans le corps du patient avec un seul injecteur relié à deux types de solutions de concentrations différentes (ou une solution de contraste et une solution saline) à un coût moindre et nécessitant moins de connections et de manipulations qui représentent toujours un risque en terme d'asepsie et d'erreurs.

Typiquement on peut choisir le pourcentage souhaité de dilution à obtenir dans le coeur (p.ex. 15%, 20%, 25% ou 30% de dilution, c'est à dire pour 25% qu'il y aura 25% de produit de contraste seulement et 75% de solution saline). Dans le cas d'une dilution à 25%, on peut choisir par exemple une phase de 1 ml de contraste suivie d'une phases de 3ml de saline, les deux phases représentant un cycle qui est répété en fonction du volume total que l'on souhaite injecter (p. ex. pour 20ml injecté on réalisera 5 cycles successifs dans l'exemple décrit). Le débit est, dans ce cas, le même pour la solution saline et la solution de contraste (p. ex. 4ml/s). Alternativement on peut aussi faire varier le débit afin d'obtenir le même effet de dilution avec un volume modifié. Ainsi dans l'exemple décrit plus haut, on peut remplacer les 3ml de la phase de solution saline par une phase de 1.5ml avec un débit réduit de moitié (dans l'exemple 2ml/s au lieu des 4ml/s). On peut aussi choisir de faire varier le débit de la solution de contraste et réaliser d'autres combinaisons ayant pour effet de modifier les rapports de contraste dans l'organe ciblé. On peut également choisir de faire varier les débit et/ou volumes respectifs de saline et de contraste à chaque cycle, par exemple de façon progressive, afin d'obtenir des images dynamiques avec des concentrations variables au cour du temps d'examen et/ou d'acquisition de l'image, selon des algorithmes pouvant revêtir toute forme mathématique utile.

Afin de réaliser un tel dispositif, on utilise de préférence un processeur capable de gérer l'alternance entre les deux réservoirs, par exemple en commandant l'ouverture et la fermeture de clamps situés entre chacun des réservoirs et le dispositif d'injection (par exemple une cassette péristaltique).

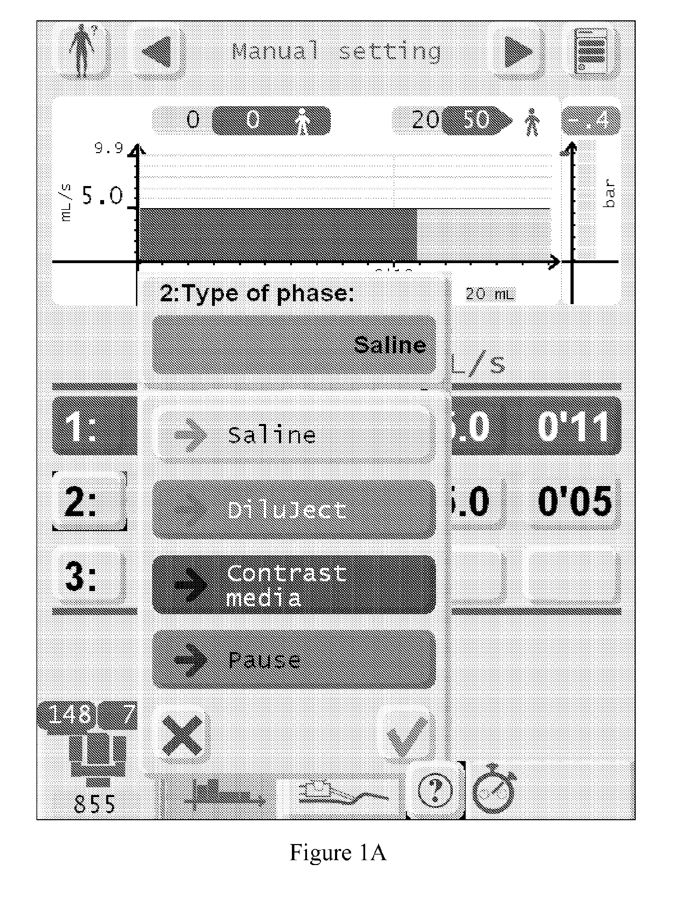

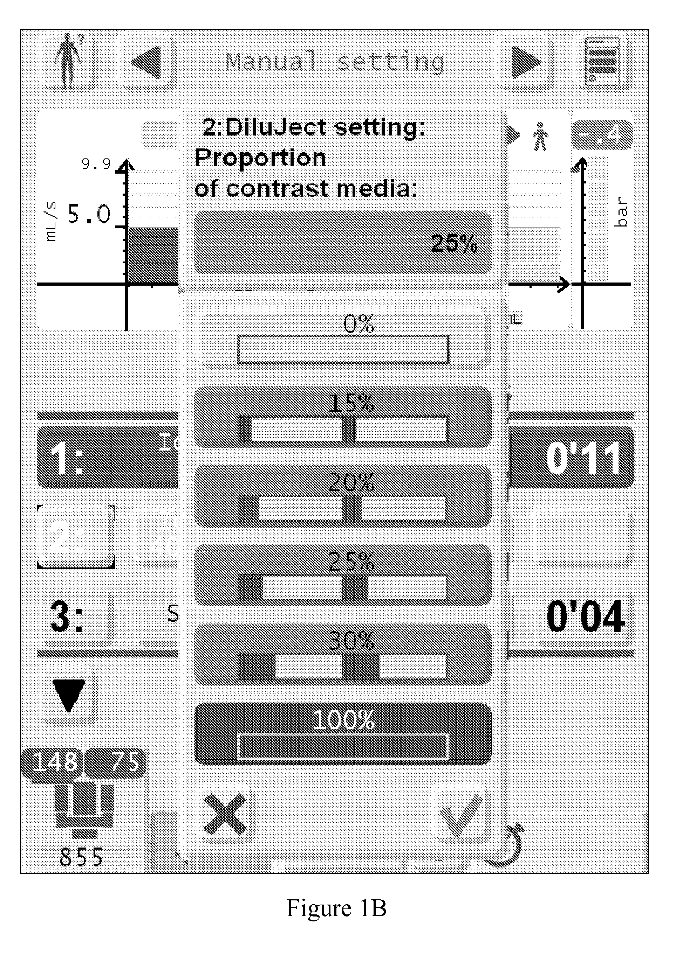

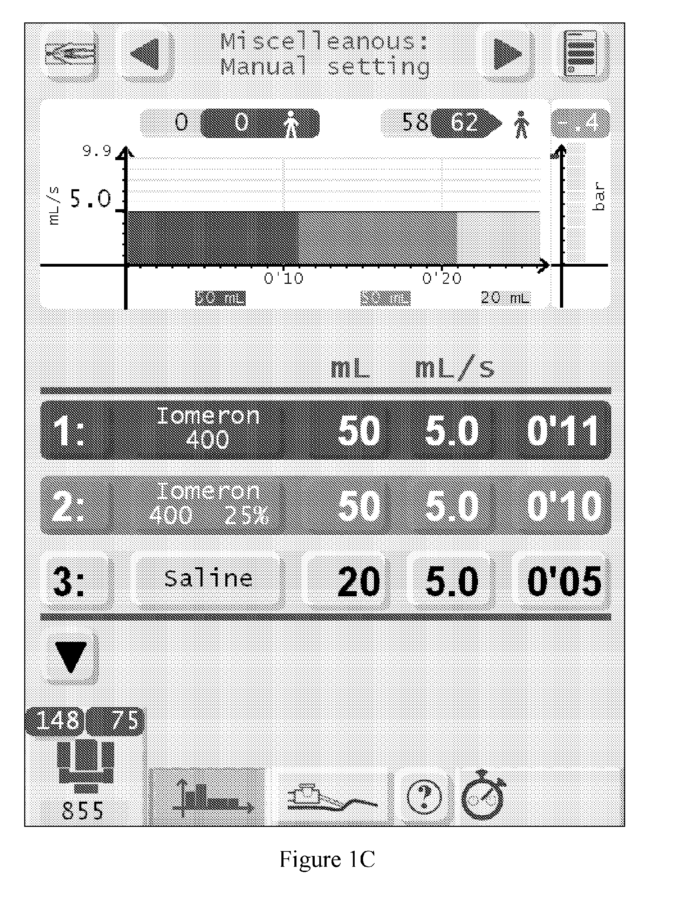

Les figures 1A, 1B et 1C présentent un exemple d'interface de programmation de l'injecteur, figurant les différentes concentrations possibles avec en Figure 1A l'option de choix entre l'injection de contraste, de saline ou de dilution « Diluject en Figure 1B les possibilités de dilution (15%, 20%, 25% ou 30%) ; et en figure 1C la phase de dilution sur un diagramme.

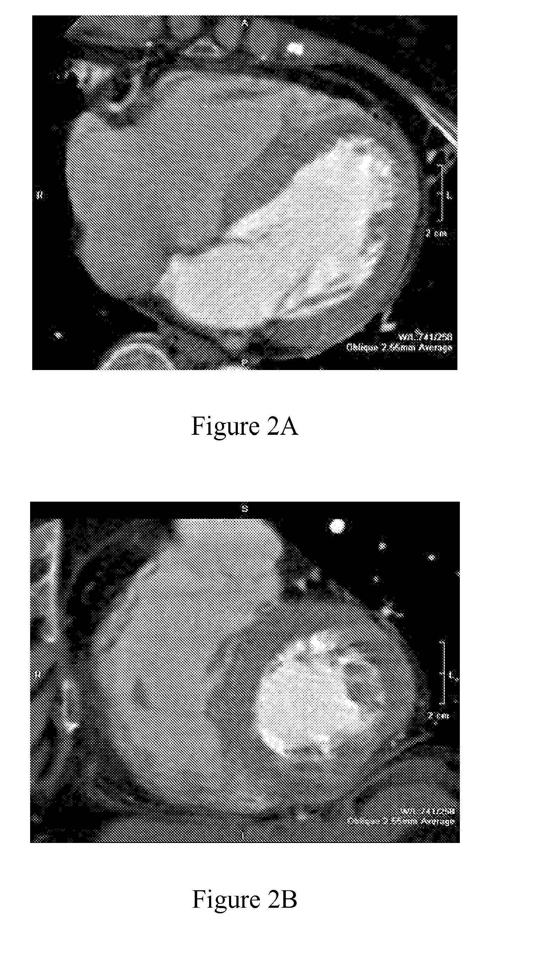

Les figures 2A et 2B représentent des images obtenues avec une dilution de 15% et un débit de 4ml/s (2A et 2B) et montrent une dilution optimale dans le coeur avec une différence, dans cet exemple, entre la concentration dans le coeur droit et le coeur gauche.

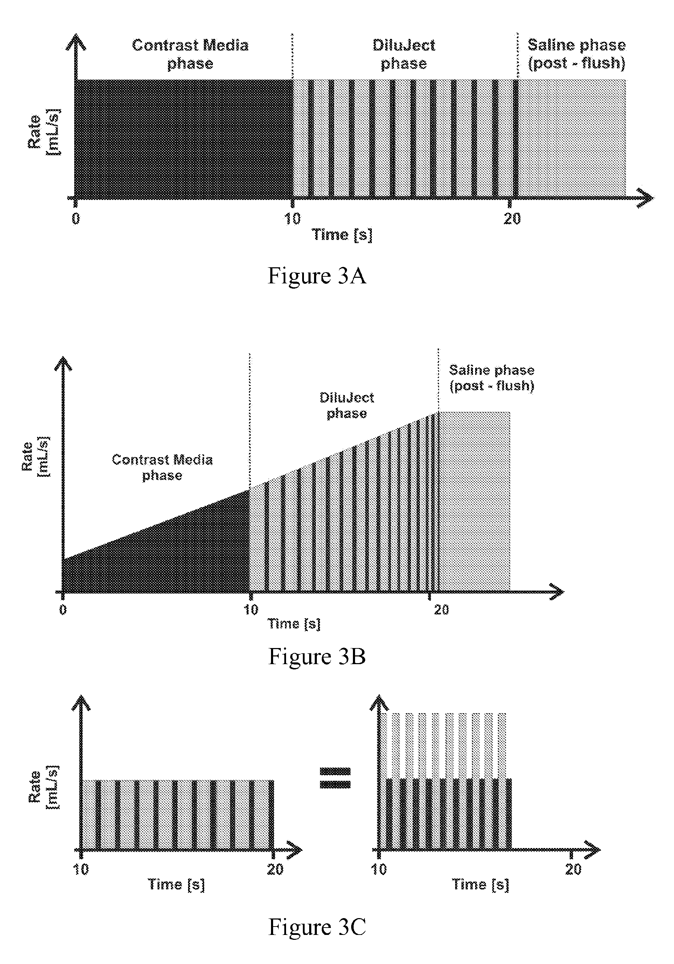

Les figures 3A-3C (3A dilution fixe, 3B dilution à débit et fréquence variable, 3C deux exemples de dilution à débits différents) représentent des exemples de cycles d'injection avec différents modes de dilution.

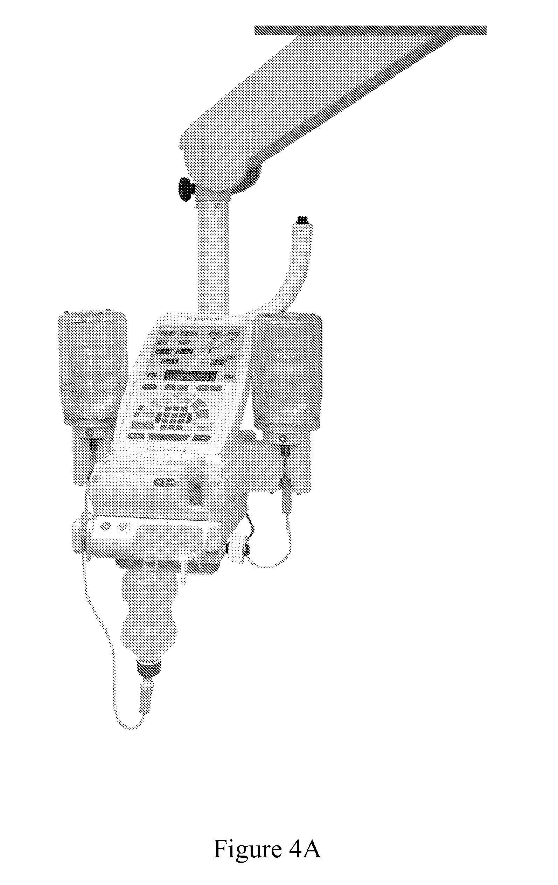

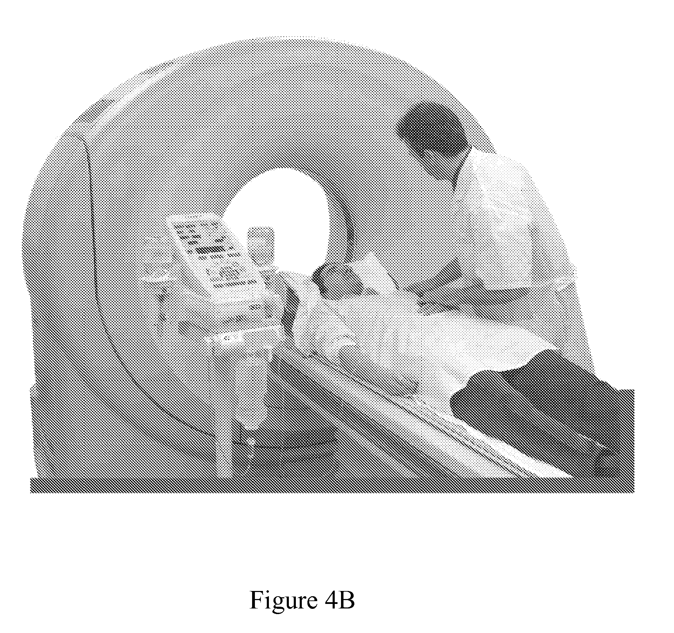

Les figures 4A et 4B représentent un injecteur permettant la réalisation de l'invention.

## Revendications

1. Dispositif médical pour l'injection de produits de contraste comprenant au moins deux réservoirs, un injecteur et un distributeur disposé de manière à mettre en communication de manière alternée lesdits réservoirs avec ledit injecteur, dispositif médical **caractérisé par le fait qu'**il comprend des moyens pour assurer ladite communication alternée à une fréquence comprise entre 0.2 et 5 Hz pendant au minimum deux cycles consécutifs.

2. Dispositif médical selon la revendication 1 dans lequel la fréquence est d'environ 1 Hz.

3. Dispositif médical selon la revendication 1 dans lequel la fréquence est variable au cours du temps.

4. Dispositif médical selon la revendication 1 à 3 adapté pour autoriser un écoulement continu compris entre 0.1 à 5 ml avec le même réservoir.

5. Dispositif médical selon l'une des revendications précédentes dans lequel un réservoir contient un produit de contraste et l'autre réservoir contient une solution saline.

6. Dispositif médical selon l'une des revendications précédentes 1 à 4 dans lequel chaque réservoir comporte un produit de contraste avec une concentration qui lui est spécifique.

7. Utilisation d'un dispositif médical selon l'une des revendications précédentes **caractérisé par le fait que** l'on alterne la mise communication des réservoirs avec l'injecteur à une fréquence comprise entre 0.2 et 5 Hz.

8. Utilisation selon la revendication 7 dans laquelle ladite fréquence est d'environ 1 Hz.

9. Utilisation selon la revendication 7 ou 8 dans laquelle on autorise un écoulement continu compris entre 0.1 à 5 ml avec le même réservoir.

10. Utilisation selon l'un des revendications précédentes 7 à 9 dans laquelle on injecte un volume inférieur à 5 ml depuis un réservoir à chaque alternance.

11. Utilisation selon l'un des revendications précédentes 7 à 10 pour l'injection de produits de contraste dans le coeur.
